# EUROPÄISCHE PATENTSCHRIFT

(11) **EP 1 351 962 B1**
(45) Veröffentlichungstag und Bekanntmachung des Hinweises auf die Patenterteilung: **16.03.2005**
(21) Anmeldenummer: 01974106.5
(22) Anmeldetag: 03.08.2001
(51) Int. Cl.: C07D 495/04

(54) **THIENOPYRIMIDINE**
THIENOPYRIMIDINE
THIENOPYRIMIDINES

(30) Priorität: 01.09.2000 DE 10042997
(43) Veröffentlichungstag der Anmeldung: 15.10.2003
(73) Patentinhaber: MERCK PATENT GmbH, 64293 Darmstadt (DE)
(72) Erfinder: JONAS, Rochus, 64291 Darmstadt (DE); EGGENWEILER, Hans-Michael, 64287 Darmstadt (DE); SCHELLING, Pierre, 64367 Mühltal (DE); CHRISTADLER, Maria, 63322 Rödermark (DE); BEIER, Norbert, 64354 Reinheim (DE)
(86) Internationale Anmeldenummer: PCT/EP2001/008998
(87) Internationale Veröffentlichungsnummer: WO 2002/018389

(56) Entgegenhaltungen:
- WO-A-98/17668
- DE-A- 19 752 952

## Beschreibung

Die Erfindung betrifft Verbindungen der Formel I 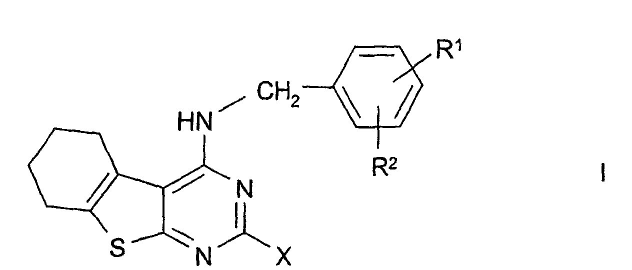 worin
- R¹, R²: jeweils unabhängig voneinander H, A, OH, OA, NO₂ oder Hal,
- R¹ und R²: zusammen auch Alkylen mit 3-5 C-Atomen, -O-CH₂-CH₂-, -CH₂-O-CH₂-, -O-CH₂-O- oder -O-CH₂-CH₂-O-,
- X: einfach durch R⁵ substituiertes R³ oder R⁴,
- R³: lineares oder verzweigtes Alkylen mit 1-10 C-Atomen, worin eine oder zwei CH₂-Gruppen durch -CH=CH-Gruppen, O, NH oder NA ersetzt sein können,
- R⁴: Cycloalkyl oder Cycloalkylalkylen mit 5-12 C-Atomen,
- R⁵: O(CH₂)ₙCOOH, O(CH₂)ₙCOOA, O(CH₂)ₙCONH₂, O(CH₂)ₙCONHA, O(CH₂)ₙCONA₂ oder O(CH₂)ₙCN, S(O)ₘ(CH₂)ₙCOOH, S(O)ₘ(CH₂)ₙCOOA, S(O)ₘ(CH₂)ₙCONH₂, S(O)ₘ(CH₂)ₙCONHA, S(O)ₘ(CH₂)ₙCONA₂ oder S(O)ₘ(CH₂)ₙCN,
- m: 0, 1 oder 2,
- n: 1 oder 2,
- A: Alkyl mit 1 bis 6 C-Atomen und
- Hal: F, Cl, Br oder I
bedeuten,
sowie deren physiologisch unbedenklichen Salze und/oder Solvate.

Pyrimidinderivate sind beispielsweise aus der WO 99/55708, EP 934321, EP 201 188 oder der WO 93/06104 bekannt.

DE-A- 197 52 952 beschreibt PDE-V Inhibitoren auf Basis von Thienopyrimidinen, ähnlish den vorliegenden Verbindungen.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze und/oder Solvate bei guter Verträglichkeit sehr wertvolle pharmakologische Eigenschaften besitzen.

Insbesondere zeigen sie eine spezifische Inhibierung der cGMP-Phosphodiesterase (PDE V).

Chinazoline mit cGMP-Phosphodiesterase hemmender Aktivität sind z.B. in J. Med. Chem. 36, 3765 (1993) und ibid. 37, 2106 (1994) beschrieben.

Die biologische Aktivität der Verbindungen der Formel I kann nach Methoden bestimmt werden, wie sie z.B in der WO 93/06104 beschrieben sind. Die Affinität der erfindungsgemäßen Verbindungen für cGMP- und cAMP-Phosphodiesterase wird durch die Ermittlung ihrer IC₅₀-Werte (Konzentration des Inhibitors, die benötigt wird, um eine 50 %ige Inhibierung der Enzymaktivität zu erreichen) bestimmt.
Zur Durchführung der Bestimmungen können nach bekannten Methoden isolierte Enzyme verwendet werden (z.B. W.J. Thompson et al., Biochem. 1971, 10, 311). Zur Durchführung der Versuche kann eine modifizierte "batch"-Methode von W.J. Thompson und M.M. Appleman (Biochem. 1979, 18, 5228) angewendet werden.

Die Verbindungen eignen sich daher zur Behandlung von Erkrankungen des Herz-Kreislaufsystems, insbesondere der Herzinsuffizienz und zur Behandlung und/oder Therapie von Potenzstörungen (erektile Dysfunktion).

Die Verbindungen eignen sich weiterhin zur Behandlung von Angina, Bluthochdruck, pulmonarem Hochdruck, congestivem Herzversagen, Atherosklerose, Bedingungen verminderter Durchgängigkeit der Herzgefäße, peripheren vaskulären Krankheiten, Schlaganfall, Bronchitis, allergischem Asthma, chronischem Asthma, allergischer Rhinitis, Glaucom, Irritable Bowel Syndrome, Tumoren, Niereninsuffizienz, Leberzirrhose und zur Behandlung weiblicher Sexualstörungen.

Die Verwendung von substituierten Pyrazolopyrimidinonen zur Behandlung von Impotenz ist z.B. in der WO 94/28902 beschrieben.

Die Verbindungen sind wirksam als Inhibitoren der Phenylephrin-induzierten Kontraktionen in Corpus cavemosum-Präparationen von Hasen.
Diese biologische Wirkung kann z.B. nach der Methode nachgewiesen werden, die von F. Holmquist et al. in J. Urol., 150, 1310-1315 (1993) beschrieben wird.
Die Inhibierung der Kontraktion, zeigt die Wirksamkeit der erfindungsgemäßen Verbindungen zur Therapie und/oder Behandlung von Potenzstörungen.

Die Verbindungen der Formel I können als Arzneimittelwirkstoffe in der Human- und Veterinärmedizin eingesetzt werden. Ferner können sie als Zwischenprodukte zur Herstellung weiterer Arzneimittelwirkstoffe eingesetzt werden.

Gegenstand der Erfindung sind dementsprechend die Verbindungen der Formel I sowie ein Verfahren zur Herstellung
von Verbindungen der Formel I nach Anspruch 1 sowie deren Salzen,
dadurch gekennzeichnet, daß man
a) eine Verbindung der Formel II 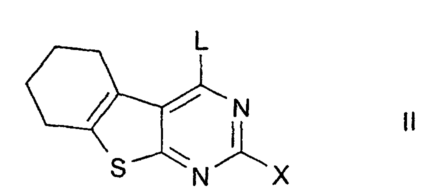 worin
   X die angegebene Bedeutung hat,
   und L Cl, Br, OH, SCH₃ oder eine reaktionsfähige veresterte OH-Gruppe bedeutet,
   mit einer Verbindung der Formel III 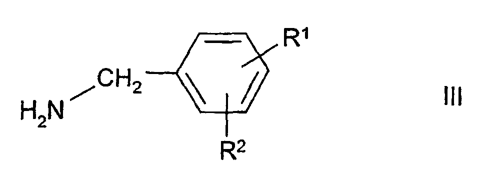 worin
   R¹ und R² die angegebenen Bedeutungen haben,
   umsetzt,
   oder
b) in einer Verbindung der Formel I einen Rest X in einen anderen Rest X umwandelt, indem man z.B. eine Estergruppe zu einer COOH-Gruppe hydrolysiert oder eine COOH-Gruppe in ein Amid oder in eine Cyangruppe umwandelt
   und/oder daß man eine Verbindung der Formel I in eines ihrer Salze überführt.

Vor- und nachstehend haben die Reste R¹, R², R³, R⁴, R⁵, X und L die bei den Formeln I, II und III angegebenen Bedeutungen, sofern nicht ausdrücklich etwas anderes angegeben ist.

A bedeutet Alkyl mit 1-6 C-Atomen.
In den vorstehenden Formeln ist Alkyl vorzugsweise unverzweigt und hat 1, 2, 3,4, 5 oder 6 C-Atome und bedeutet vorzugsweise Methyl, Ethyl oder Propyl, weiterhin bevorzugt Isopropyl, Butyl, Isobutyl, sek.-Butyl oder tert.-Butyl, aber auch n-Pentyl, Neopentyl, Isopentyl oder Hexyl.
A bedeutet weiterhin Alkenyl mit 2-6 C-Atomen, z.B. Vinyl oder Propenyl. A bedeutet ferner einen halogenierten Alkylrest, wie z.B. Trifluormethyl.

X bedeutet einen einfach durch R⁵ substituierten R³- oder R⁴-Rest.

R³ bedeutet einen linearen oder verzweigten Alkylenrest mit 1-10 C-Atomen, wobei der Alkylenrest vorzugsweise z.B. Methylen, Ethylen, Propylen, Isopropylen, Butylen, Isobutylen, sek.-Butylen, Pentylen, 1-, 2- oder 3-Methylbutylen, 1,1- , 1,2- oder 2,2-Dimethylpropylen, 1-Ethylpropylen, Hexylen, 1- , 2- , 3- oder 4-Methylpentylen, 1,1- , 1,2- , 1,3- , 2,2- , 2,3- oder 3,3-Dimethylbutylen, 1- oder 2-Ethylbutylen, 1-Ethyl-1-methylpropylen, 1-Ethyl-2-methylpropylen, 1,1,2- oder 1,2,2-Trimethylpropylen, lineares oder verzweigtes Heptylen, Octylen, Nonylen oder Decylen bedeutet.
R³ bedeutet ferner z.B. But-2-en-ylen oder Hex-3-en-ylen.
Ganz besonders bevorzugt ist Methylen, Ethylen, Propylen oder Butylen.

R⁴ bedeutet Cycloalkylalkylen mit 5-12 C-Atomen, vorzugsweise z.B. Cycclopentylmethylen, Cyclohexylmethylen, Cyclohexylethylen, Cyclohexylpropylen oder Cyclohexylbutylen.
R⁴ bedeutet auch Cycloalkyl mit vorzugsweise mit 5-7 C-Atomen. Cycloalkyl bedeutet z.B. Cyclopentyl, Cyclohexyl oder Cycloheptyl.

R⁵ bedeutet vorzugsweise z.B. OCH₂COOH, OCH₂COOA, S(O)ₘCH₂COOH oder S(O)ₘCH₂COOA.

Hal bedeutet vorzugsweise F, Cl oder Br, aber auch I.

Die Reste R¹ und R² können gleich oder verschieden sein und stehen vorzugsweise in der 3- oder 4-Position des Phenylrings. Sie bedeuten beispielsweise jeweils unabhängig voneinander H, OH, Alkyl, F, Cl, Br oder l oder zusammen Alkylen, wie z.B. Propylen, Butylen oder Pentylen, femer Ethylenoxy, Methylendioxy oder Ethylendioxy. Bevorzugt stehen sie auch jeweils für Alkoxy, wie z.B. für Methoxy, Ethoxy oder Propoxy.

Unter Solvaten versteht man die Hydrate oder z.B. Alkoholate.

Für die gesamte Erfindung gilt, daß sämtliche Reste, die mehrfach auftreten, gleich oder verschieden sein können, d.h. unabhängig voneinander sind.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln la bis If ausgedrückt werden, die der Formel I entsprechen und worin die nicht näher bezeichneten Reste die bei der Formel I angegebene Bedeutung haben, worin jedoch
in la
   - X: durch O(CH₂)ₙCOOH, O(CH₂)ₙCOOA, O(CH₂)ₙCONH₂, O(CH₂)ₙCONHA, O(CH₂)ₙCONA₂ o- der O(CH₂)ₙCN, S(O)ₘ(CH₂)ₙCOOH, S(O)ₘ(CH₂)ₙCOOA, S(O)m(CH₂)ₙCONH₂, S(O)ₘ(CH₂)ₙCONHA, S(O)ₘ(CH₂)ₙCONA₂ oder S(O)ₘ(CH₂)ₙCN substituiertes R³ bedeutet;
in Ib
   - R¹, R²: jeweils unabhängig voneinander Hal, OH oder OA,
   - X: durch O(CH₂)ₙCOOH, O(CH₂)ₙCOOA, S(O)ₘ(CH₂)ₙCOOH, S(O)ₘ(CH₂)ₙCOOA, substituiertes R³
   bedeuten;
in lc
   - R¹, R²: jeweils unabhängig voneinander Hal, OH oder OA,
   - X: durch O(CH₂)ₙCOOH, O(CH₂)ₙCOOA, S(O)ₘ(CH₂)ₙCOOH, S(O)ₘ(CH₂)ₙCOOA, substituiertes R³,
   - R³: Methylen, Ethylen oder Propylen bedeuten;
in ld
   - R¹, R²: jeweils unabhängig voneinander Hal, OH oder OA,
   - R¹ und R²: zusammen Alkylen mit 3-5 C-Atomen, -O-CH₂-CH₂-, -O-CH₂-O- oder -O-CH₂-CH₂-O,
   - X: durch O(CH₂)ₙCOOH, O(CH₂)ₙCOOA, S(O)ₘ(CH₂)ₙCOOH, S(O)ₘ(CH₂)ₙCOOA, substituiertes R³,
   - R³: Methylen, Ethylen oder Propylen bedeuten;
in le
   - R¹, R²: jeweils unabhängig voneinander H, Hal, A, NO₂, OH oder OA,
   - R¹ und R²: zusammen Alkylen mit 3-5 C-Atomen, -O-CH₂CH₂-, -O-CH₂-O- oder -O-CH₂-CH₂O-,
   - X: durch O(CH₂)ₙCOOH, O(CH₂)ₙCOOA, S(O)ₘ(CH₂)ₙCOOH, S(O)ₘ(CH₂)ₙCOOA, substituiertes R³,
   - R³: Methylen, Ethylen oder Propylen,
   - A: Alkyl mit 1-6 C-Atomen oder CF₃ bedeuten;
in If
   - R¹, R²: jeweils unabhängig voneinander H, Hal, A, NO₂, OH oder OA,
   - R¹ und R²: zusammen -O-CH₂-O- oder -O-CH₂-CH₂-O-,
   - X: durch O(CH₂)ₙCOOH, O(CH₂)ₙCOOA, S(O)ₘ(CH₂)ₙCOOH, S(O)ₘ(CH₂)ₙCOOA, substituiertes R³,
   - R³: Methylen, Ethylen oder Propylen,
   - A: Alkyl mit 1-6 C-Atomen oder CF₃ bedeuten;
sowie deren physiologisch unbedenklichen Salze und/oder Solvate.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart), beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

In den Verbindungen der Formeln II oder III haben R¹, R² und X die angegebenen Bedeutungen, insbesondere die angegebenen bevorzugten Bedeutungen.

Falls L eine reaktionsfähige veresterte OH-Gruppe bedeutet, so ist diese vorzugsweise Alkylsulfonyloxy mit 1-6 C-Atomen (bevorzugt Methylsulfonyloxy) oder Arylsulfonyloxy mit 6-10 C-Atomen (bevorzugt Phenyl- oder p-Tolylsulfonyloxy, ferner auch 2-Naphthalinsulfonyloxy).

Die Verbindungen der Formel I können vorzugsweise erhalten werden, indem man Verbindungen der Formel II mit Verbindungen der Formel III umsetzt.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.
Andererseits ist es möglich, die Reaktion stufenweise durchzuführen.

Die Ausgangsverbindungen der Formel II und III sind in der Regel bekannt. Sind sie nicht bekannt, so können sie nach an sich bekannten Methoden hergestellt werden.
Verbindungen der Formel II können z.B. durch Umsetzung mit POCl₃ aus den entsprechenden Hydroxypyrimidinen erhalten werden, die aus Thiophenderivaten und CN-substituierten Alkylencarbonsäureestem aufgebaut werden (Eur. J. Med. Chem. 23, 453 (1988)).
Die Darstellung der Hydroxypyrimidine erfolgt entweder durch Dehydrierung entsprechender Tetrahydrobenzthienopyrimidinverbindungen oder nach der für die Herstellung von Pyrimidinderivaten üblichen Cyclisierung von 2-Aminobenzthiophen-3-carbonsäure-derivaten mit Aldehyden oder Nitrilen (z.B. Houben Weyl E9b/2).

Im einzelnen erfolgt die Umsetzung der Verbindungen der Formel II mit den Verbindungen der Formel III in Gegenwart oder Abwesenheit eines inerten Lösungsmittels bei Temperaturen zwischen etwa -20 und etwa 150°, vorzugsweise zwischen 20 und 100°.

Der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylamin, Pyridin oder Chinolin oder eines Überschusses der Aminkomponente kann günstig sein.

Als inerte Lösungsmittel eignen sich z.B. Kohlenwasserstoffe wie Hexan, Petrolether, Benzol, Toluol oder Xylol; chlorierte Kohlenwassertoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid, N-Methylpyrrolidon oder Dimethylformamid (DMF); Nitrile wie Acetonitril; Sulfoxide wie Dimethylsulfoxid (DMSO); Nitroverbindungen wie Nitromethan oder Nitrobenzol; Ester wie Ethylacetat oder Gemische der genannten Lösungsmittel.

Es ist femer möglich, in einer Verbindung der Formel I einen Rest X in einen anderen Rest X umzuwandeln, z.B. indem man einen Ester oder eine Cyangruppe zu einer COOH-Gruppe hydrolysiert.
Estergruppen können z.B. mit NaOH oder KOH in Wasser, Wasser-THF oder Wasser-Dioxan bei Temperaturen zwischen 0 und 100° verseift werden.
Carbonsäuren können z.B. mit Thionylchlorid in die entsprechenden Carbonsäurechloride und diese in Carbonsäureamide umgewandelt werden. Durch Wasserabspaltung in bekannter Weise erhält man aus diesen Carbonitrile.

Eine Säure der Formel I kann mit einer Base in das zugehörige Säureadditionssalz übergeführt werden, beispielsweise durch Umsetzung äquivalenter Mengen der Säure und der Base in einem inerten Lösungsmittel wie Ethanol und anschließendes Eindampfen. Für diese Umsetzung kommen insbesondere Basen in Frage, die physiologisch unbedenkliche Salze liefern.
So kann die Säure der Formel I mit einer Base (z.B. Natrium- oder Kaliumhydroxid oder -carbonat) in das entsprechende Metall-, insbesondere Alkalimetall- oder Erdalkalimetall-, oder in das entsprechende Ammoniumsalz umgewandelt werden.
Für diese Umsetzung kommen insbesondere auch organische Basen in Frage, die physiologisch unbedenkliche Salze liefern, wie z.B. Ethanolamin.

Andererseits kann eine Base der Formel I mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bemsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und /oder Aufreinigung der Verbindungen der Formel 1 verwendet werden.

Gegenstand der Erfindung ist ferner die Verwendung der Verbindungen der Formel I und/oder ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind auch Arzneimittel der Formel I und ihre physiologisch unbedenklichen Salze als Phosphodiesterase V-Hemmer.

Gegenstand der Erfindung sind ferner pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z.B. ein oder mehrere Vitamine.

Die Verbindungen der Formel I und ihre physiologisch unbedenklichen Salze können bei der Bekämpfung von Krankheiten, bei denen eine Erhöhung des cGMP(cyclo-Guanosin-monophosphat)-Spiegels zu Entzündungshemmung oder -verhinderung und Muskelentspannung führt, eingesetzt werden. Besondere Verwendung können die erfindungsgemäßen Verbindungen bei der Behandlung von Krankheiten des Herz-Kreislaufsystems und zur Behandlung und/oder Therapie von Potenzstörungen finden.

Gegenstand der Erfindung ist die Verwendung der Verbindungen der Formel I sowie deren physiologisch unbedenklichen Salze und/oder Solvate zur Herstellung eines Arzneimittels zur Behandlung von Angina, Bluthochdruck, pulmonarem Hochdruck, congestivem Herzversagen, Atherosklerose, Bedingungen verminderter Durchgängigkeit der Herzgefäße, peripheren vaskulären Krankheiten, Schlaganfall, Bronchitis, allergischem Asthma, chronischem Asthma, allergischer Rhinitis, Glaucom, Irritable Bowel Syndrome, Tumoren, Niereninsuffizienz, Leberzirrhose und zur Behandlung weiblicher Sexualstörungen.

Dabei werden die Substanzen in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation.

Massenspektrometrie (MS): El (Elektronenstoß-Ionisation) M⁺
FAB (Fast Atom Bombardment) (M+H)⁺

### Beispiel 1

Zu einer Suspension von 115 g Schwefel und 300 ml Methylcyanacetat in 500 ml Methanol werden bei Raumtemperatur 357 ml Cyclohexanon zugetropft. Danach gibt man langsam 350 ml Diethylamin zu, wobei die Temperatur bei max. 50° gehalten wird. Man rührt 12 Stunden nach, kühlt auf 4° ab, trennt die Kristalle ab und wäscht mit eiskaltem Methanol. Nach Trocknen erhält man 580 g 2-Amino-4,5,6,7-tetrahydro-benzo[*b*]thiophen-3-carbonsäure-methylester ("AA"), F. 130°.

Zu einer Lösung von 106 g "AA" in 600 ml Dioxan gibt man 40 ml Chloracetonitril und leitet bei 40-50° 3 Stunden lang unter Rühren HCl ein. Man rührt 2 Stunden nach, entfernt das Lösungsmittel und arbeitet wie üblich auf. Man erhält 125 g 2-Chlormethyl-5,6,7,8-tetrahydro-3H-benzo[4,5]-thieno[2,3-*d*]pyrimidin-4-on ("AB"), F. 285-286°.

Eine Lösung von 5,0 g "AB" und 2,8 g Glycolsäure-butylester in 100 ml THF wird mit 1,7 g Natriumhydrid (60%ige Suspension) versetzt und 3 Stunden unter Rückfluß erhitzt. Man entfernt das Lösungsmittel, arbeitet wie üblich auf und erhält 5,0 g (4-Oxo-3,4,5,6,7,8-hexahydro-benzo[4,5]-thieno[2,3-*d*]pyrimidin-2-ylmethoxy)-essigsäure-butylester ("AC").

Eine Lösung von 5,0 g "AC" in 50 ml Thionylchlorid wird mit 1 ml DMF versetzt und 2 Stunden bei 45° gerührt. Nach Entfernen der Lösungsmittel wird wie üblich aufgearbeitet und man erhält 4,5 g (4-Chlor-5,6,7,8-tetrahydro-benzo[4,5]thieno[2,3-*d*]pyrimidin-2-ylmethoxy)-essigsäurebutylester ("AD").

### Beispiel 2

Analog wird durch Umsetzung von "AA" mit Chlorpropionitril und weiterer Reaktion analog Beispiel 1 die Verbindung (4-Chlor-5,6,7,8-tetrahydrobenzo[4,5]thieno[2,3-*d*]pyrimidin-2-ylethoxy)-essigsäure-butylester ("AE") erhalten.

### Beispiel 3

Eine Lösung von 4,5 g "AD" und 4,5 g 3-Chlor-4-Methoxybenzylamin in 30 ml 1-Methyl-pyrrolidon wird 1 Stunde bei 100° erhitzt. Man entfernt das Lösungsmittel, arbeitet wie üblich auf und erhält 1,8 g [4-(3-Chlor-4-methoxybenzylamino)-5,6,7,8-tetrahydro-benzo[4,5]thieno[2,3-*d*]pyrimidin-2-ylmethoxy]-essigsäure-butylester.

Analog erhält man aus "AE" die Verbindung [4-(3-Chlor-4-methoxybenzylamino)-5,6,7,8-tetrahydro-benzo[4,5]thieno[2,3-*d*]pyrimidin-2-ylethoxy]-essigsäure-butylester.

Analog erhält man durch Umsetzung von
3,4-Methylendioxybenzylamin,
3,4-Dimethoxybenzylamin,
4-Methoxybenzylamin,
3,4-Dichlorbenzylamin,
4-Chlorbenzylamin,
4-Methylbenzylamin,
4-Fluorbenzylamin,
Benzylamin,
3-Chlor-4-nitrobenzylamin,
2,4-Dichlorbenzylamin,
2-Chlor-4-fluorbenzylamin,
3-Fluorbenzylamin,
2-Methoxybenzylamin,
2-Chlorbenzylamin,
3,5-Di-(trifluormethoxy)benzylamin,
mit "AD" bzw. "AE" die nachstehenden Verbindungen
[4-(3,4-Methylendioxy-benzylamino)-5,6,7,8-tetrahydro-benzo[4,5]-thieno[2,3-*d*]pyrimidin-2-ylmethoxy]-essigsäure-butylester,
[4-(3,4-Dimethoxy-benzylamino)-5,6,7,8-tetrahydro-benzo[4,5]-thieno[2,3-*d*]pyrimidin-2-ylmethoxy]-essigsäure-butylester,
[4-(4-Methoxybenzylamino)-5,6,7,8-tetrahydro-benzo[4,5]thieno[2,3-*d*]pyrimidin-2-ylmethoxy]-essigsäure-butylester,
[4-(3,4-Dichlorbenzylamino)-5,6,7,8-tetrahydro-benzo[4,5]thieno[2,3-*d*]pyrimidin-2-ylmethoxy]-essigsäure-butylester,
[4-(4-Chlorbenzylamino)-5,6,7,8-tetrahydro-benzo[4,5)thieno[2,3-*d*]pyrimidin-2-ylmethoxy]-essigsäure-butylester,
[4-(4-Methylbenzylamino)-5,6,7,8-tetrahydro-benzo[4,5]thieno[2,3-*d*]pyrimidin-2-ylmethoxy]-essigsäure-butylester,
[4-(4-Fluorbenzylamino)-5,6,7,8-tetrahydro-benzo[4,5]thieno[2,3-*d*]pyrimidin-2-ylmethoxy]-essigsäure-butylester,
(4-Benzylamino-5,6,7,8-tetrahydro-benzo[4,5]thieno[2,3-*d*]pyrimidin-2-ylmethoxy)-essigsäure-butylester,
[4-(3-Chlor-4-nitrobenzylamino)-5,6,7,8-tetrahydro-benzo[4,5]-thieno[2,3-d]pyrimidin-2-ylmethoxy]-essigsäure-butylester,
[4-(2,4-Dichlorbenzylamino)-5,6,7,8-tetrahydro-benzo[4,5]thieno[2,3-*d*]pyrimidin-2-ylmethoxy]-essigsäure-butylester,
[4-(2-Chlor-4-fluorbenzylamino)-5,6,7,8-tetrahydro-benzo[4,5]-thieno[2,3-*d*]pyrimidin-2-ylmethoxy]-essigsäure-butylester,
[4-(3-Fluorbenzylamino)-5,6,7,8-tetrahydro-benzo[4,5]thieno[2,3-*d*]pyrimidin-2-ylmethoxy]-essigsäure-butylester,
[4-(2-Methoxybenzylamino)-5,6,7,8-tetrahydro-benzo[4,5]thieno[2,3-*d*]pyrimidin-2-ylmethoxy]-essigsäure-butylester,
[4-(2-Chlorbenzylamino)-5,6,7,8-tetrahydro-benzo[4,5]thieno[2,3-*d*]pyrimidin-2-ylmethoxy]-essigsäure-butylester,
{4-[3,5-Di-(trifluormethoxy)benzylamino]-5,6,7,8-tetrahydrobenzo[4,5]thieno[2,3-*d*]pyrimidin-2-ylmethoxy)-essigsäure-butylester,
[4-(3,4-Methylendioxy-benzylamino)-5,6,7,8-tetrahydro-benzo[4,5]-thieno[2,3-*d*]pyrimidin-2-ylethoxy]-essigsäure-butylester,
[4-(3,4-Dimethoxy-benzylamino)-5,6,7,8-tetrahydro-benzo[4,5]-thieno[2,3-*d*]pyrimidin-2-ylethoxy]-essigsäure-butylester,
[4-(4-Methoxybenzylamino)-5,6,7,8-tetrahydro-benzo[4,5]thieno[2,3-*d*]pyrimidin-2-ylethoxy]-essigsäure-butylester,
[4-(3,4-Dichlorbenzylamino)-5,6,7,8-tetrahydro-benzo[4,5]thieno[2,3-*d*]pyrimidin-2-ylethoxy]-essigsäure-butylester,
[4-(4-Chlorbenzylamino)-5,6,7,8-tetrahydro-benzo[4,5]thieno[2,3-*d*]pyrimidin-2-ylethoxy]-essigsäure-butylester,
[4-(4-Methylbenzylamino)-5,6,7,8-tetrahydro-benzo[4,5]thieno[2,3-*d*]pyrimidin-2-ylethoxy]-essigsäure-butylester,
[4-(4-Fluorbenzylamino)-5,6,7,8-tetrahydro-benzo[4,5]thieno[2,3-*d*]pyrimidin-2-ylethoxy]-essigsäure-butylester,
(4-Benzylamino-5,6,7,8-tetrahydro-benzo[4,5]thieno[2,3-d]pyrimidin-2-ylethoxy)-essigsäure-butylester,
[4-(3-Chlor-4-nitrobenzylamino)-5,6,7,8-tetrahydro-benzo[4,5]-thieno[2,3-*d*]pyrimidin-2-ylethoxy]-essigsäure-butylester,
[4-(2,4-Dichlorbenzylamino)-5,6,7,8-tetrahydro-benzo[4,5]thieno[2,3-*d*]pyrimidin-2-ylethoxy]-essigsäure-butylester,
[4-(2-Chlor-4-fluorbenzylamino)-5,6,7,8-tetrahydro-benzo[4,5]-thieno[2,3-*d*]pyrimidin-2-ylethoxy]-essigsäure-butylester,
[4-(3-Fluorbenzylamino)-5,6,7,8-tetrahydro-benzo[4,5]thieno[2,3-*d*]pyrimidin-2-ylethoxy]-essigsäure-butylester,
[4-(2-Methoxybenzylamino)-5,6,7,8-tetrahydro-benzo[4,5]thieno[2,3-*d*]pyrimidin-2-ylethoxy]-essigsäure-butylester,
[4-(2-Chlorbenzylamino)-5,6,7,8-tetrahydro-benzo[4,5]thieno[2,3-*d*]pyrimidin-2-ylethoxy]-essigsäure-butylester,
{4-[3,5-Di-(trifluormethoxy)benzylamino]-5,6,7,8-tetrahydrobenzo[4,5]thieno[2,3-*d*]pyrimidin-2-ylethoxy}-essigsäure-butylester.

### Beispiel 4

Analog Beispiel 1 erhält man durch Umsetzung von "AB" mit Ethylthioglycolat die Verbindung (4-Oxo-3,4,5,6,7,8-hexahydro-benzo[4,5]thieno[2,3-*d*]pyrimidin-2-ylmethylsulfanyl)-essigsäure-ethylester ("AF"), F. 172°.

Eine Mischung von 4,0 g "AF", 50 ml Phosphorylchlorid und 1 ml N-Ethyldiisopropylamin wird 2 Stunden bei 90° gerührt. Nach Entfernen des Phosphorylchlorids wird wie üblich aufgearbeitet. Man erhält 2,5 g (4-Chlor-5,6,7,8-tetrahydro-benzo[4,5]thieno[2,3-*d*]pyrimidin-2-ylmethylsulfanyl)-essigsäure-ethylester ("AG").

Analog Beispiel 3 erhält man aus "AG" die nachstehenden Benzylaminderivate
[4-(3-Chlor-4-methoxy-benzylamino)-5,6,7,8-tetrahydro-benzo[4,5]-thieno[2,3-*d*]pyrimidin-2-ylmethylsulfanyl]-essigsäure-ethylester, F. 98-99°;
[4-(3,4-Methylendioxy-benzylamino)-5,6,7,8-tetrahydro-benzo[4,5]-thieno[2,3-*d*]pyrimidin-2-ylmethylsulfanyl]-essigsäure-ethylester,
[4-(3,4-Dimethoxy-benzylamino)-5,6,7,8-tetrahydro-benzo[4,5]-thieno[2,3-*d*]pyrimidin-2-ylmethylsulfanyl]-essigsäure-ethylester,
[4-(4-Methoxybenzylamino)-5,6,7,8-tetrahydro-benzo[4,5]thieno[2,3-*d*]pyrimidin-2-ylmethylsulfanyl]-essigsäure-ethylester,
[4-(3,4-Dichlorbenzylamino)-5,6,7,8-tetrahydro-benzo[4,5]thieno[2,3-*d*]pyrimidin-2-ylmethylsulfanyl]-essigsäure-ethylester,
[4-(4-Chlorbenzylamino)-5,6,7,8-tetrahydro-benzo[4,5]thieno[2,3-*d*]pyrimidin-2-ylmethylsulfanyl]-essigsäure-ethylester,
[4-(4-Methylbenzylamino)-5,6,7,8-tetrahydro-benzo[4,5]thieno[2,3-*d*]pyrimidin-2-ylmethylsulfanyl]-essigsäure-ethylester,
[4-(4-Fluorbenzylamino)-5,6,7,8-tetrahydro-benzo[4,5]thieno[2,3-*d*]pyrimidin-2-ylmethylsulfanyl]-essigsäure-ethylester,
(4-Benzylamino-5,6,7,8-tetrahydro-benzo[4,5]thieno[2,3-*d*]pyrimidin-2-ylmethylsulfanyl)-essigsäure-ethylester,
[4-(3-Chlor-4-nitrobenzylamino)-5,6,7,8-tetrahydro-benzo[4,5]-thieno[2,3-*d*]pyrimidin-2-ylmethylsulfanyl]-essigsäure-ethylester,
[4-(2,4-Dichlorbenzylamino)-5,6,7,8-tetrahydro-benzo[4,5]thieno[2,3-*d*]pyrimidin-2-ylmethylsulfanyl]-essigsäure-ethylester,
[4-(2-Chlor-4-fluorbenzylamino)-5,6,7,8-tetrahydro-benzo[4,5]-thieno[2,3-*d*]pyrimidin-2-ylmethylsulfanyl]-essigsäure-ethylester,
[4-(3-Fluorbenzylamino)-5,6,7,8-tetrahydro-benzo[4,5]thieno[2,3-*d*]pyrimidin-2-ylmethylsulfanyl]-essigsäure-ethylester,
[4-(2-Methoxybenzylamino)-5,6,7,8-tetrahydro-benzo[4,5]thieno[2,3-*d*]pyrimidin-2-ylmethylsulfanyl]-essigsäure-ethylester,
[4-(2-Chlorbenzylamino)-5,6,7,8-tetrahydro-benzo[4,5]thieno[2,3-*d*]pyrimidin-2-ylmethylsulfanyl]-essigsäure-ethytester,
{4-[3,5-Di-(trifluormethoxy)benzylamino]-5,6,7,8-tetrahydrobenzo[4,5]thieno[2,3-*d*]pyrimidin-2-ylmethylsulfanyl}-essigsäure-ethylester.

### Beispiel 5

Zu einer Lösung von 2,0 g [4-(3-Chlor-4-methoxy-benzylamino)-5,6,7,8-tetrahydro-benzo[4,5]thieno[2,3-*d*]pyrimidin-2-ylmethylsulfanyl]-essigsäureethylester in 50 ml Eisessig gibt man 1,1 g Wasserstoffperoxid (30%ig) und rührt 3 Stunden bei Raumtemperatur.
Man arbeitet wie üblich auf und erhält 1,7 g [4-(3-Chlor-4-methoxybenzylamino)-5,6,7,8-tetrahydro-benzo[4,5]thieno[2,3-*d*]pyrimidin-2-ylmethylsulfinyl]-essigsäure-ethylester, F. 158-160°.

Analog erhält man aus den in Beispiel 4 erhaltenen Sulfanylderivaten die nachstehenden Verbindungen
[4-(3,4-Methylendioxy-benzylamino)-5,6,7,8-tetrahydro-benzo[4,5]-thieno[2,3-*d*]pyrimidin-2-ylmethylsulfinyl]-essigsäure-ethylester,
[4-(3,4-Dimethoxy-benzylamino)-5,6,7,8-tetrahydro-benzo[4,5]-thieno[2,3-*d*]pyrimidin-2-ylmethylsulfinyl]-essigsäure-ethylester,
[4-(4-Methoxybenzylamino)-5,6,7,8-tetrahydro-benzo[4,5]thieno[2,3-*d*]pyrimidin-2-ylmethylsulfinyl]-essigsäure-ethylester,
[4-(3,4-Dichlorbenzylamino)-5,6,7,8-tetrahydro-benzo[4,5]thieno[2,3-*d*]pyrimidin-2-ylmethylsulfinyl]-essigsäure-ethylester,
[4-(4-Chlorbenzylamino)-5,6,7,8-tetrahydro-benzo[4,5]thieno[2,3-*d*]pyrimidin-2-ylmethylsulfinyl]-essigsäure-ethylester,
[4-(4-Methylbenzylamino)-5,6,7,8-tetrahydro-benzo[4,5]thieno[2,3-*d*]pyrimidin-2-ylmethylsulfinyl]-essigsäure-ethylester,
[4-(4-Fluorbenzylamino)-5,6,7,8-tetrahydro-benzo[4,5]thieno[2,3-*d*]pyrimidin-2-ylmethylsulfinyl]-essigsäure-ethylester,
(4-Benzylamino-5,6,7,8-tetrahydro-benzo[4,5]thieno[2,3-*d*]pyrimidin-2-ylmethylsulfinyl)-essigsäure-ethylester,
[4-(3-Chlor-4-nitrobenzylamino)-5,6,7,8-tetrahydro-benzo[4,5]-thieno[2,3-*d*]pyrimidin-2-ylmethylsulfinyl]-essigsäure-ethylester,
[4-(2,4-Dichlorbenzylamino)-5,6,7,8-tetrahydro-benzo[4,5]thieno[2,3-*d*]pyrimidin-2-ylmethylsulfinyl]-essigsäure-ethylester,
[4-(2-Chlor-4-fluorbenzylamino)-5,6,7,8-tetrahydro-benzo[4,5]-thieno[2,3-*d*]pyrimidin-2-ylmethylsulfinyl]-essigsäure-ethylester,
[4-(3-Fluorbenzylamino)-5,6,7,8-tetrahydro-benzo[4,5]thieno[2,3-*d*]pyrimidin-2-ylmethylsulfinyl]-essigsäure-ethylester,
[4-(2-Methoxybenzylamino)-5,6,7,8-tetrahydro-benzo[4,5]thieno[2,3-*d*]pyrimidin-2-ylmethylsulfinyl]-essigsäure-ethylester,
[4-(2-Chlorbenzylamino)-5,6,7,8-tetrahydro-benzo[4,5]thieno[2,3-*d*]pyrimidin-2-ylmethylsulfinyl]-essigsäure-ethylester,
{4-[3,5-Di-(trifluormethoxy)benzylamino]-5,6,7,8-tetrahydrobenzo[4,5]thieno[2,3-*d*]pyrimidin-2-ylmethylsulfinyl}-essigsäure-ethylester.

### Beispiel 6

Eine Lösung von 1,8 g [4-(3-Chlor-4-methoxy-benzylamino)-5,6,7,8-tetrahydro-benzoj4,5]thieno[2,3-d)pyrimidin-2-ylmethoxy]-essigsäurebutylester in 60 ml Ethylenglycolmonoethylether und 20 ml 2M NaOH wird 30 Minuten im Dampfbad gerührt.
Man arbeitet wie üblich auf und erhält 1,7 g [4-(3-Chlor-4-methoxybenzylamino)-5,6,7,8-tetrahydro-benzo[4,5]thieno[2,3-*d*]pyrimidin-2-ylmethoxy]-essigsäure, F. 136-137°.

Die so erhaltene Verbindung wird in 20 ml Isopropanol und 0,3 g Ethanolamin heiß gelöst, abgekühlt und mit Ether versetzt. Die ausgefallenen Kristalle werden abgetrennt und man erhält 1,7 g [4-(3-Chlor-4-methoxybenzylamino)-5,6,7,8-tetrahydro-benzo[4,5]thieno[2,3-*d*]pyrimidin-2-ylmethoxy]-essigsäure, Ethanolaminsalz, F. 148-149°.

Analog erhält man aus den in Beispiel 3 erhaltenen Estern die nachstehenden Carbonsäurederivate
[4-(3,4-Methylendioxy-benzylamino)-5,6,7,8-tetrahydro-benzo[4,5]-thieno[2,3-*d*]pyrimidin-2-ylmethoxy]-essigsäure,
[4-(3,4-Dimethoxy-benzylamino)-5,6,7,8-tetrahydro-benzo[4,5]-thieno[2,3-*d*]pyrimidin-2-ylmethoxy]-essigsäure,
[4-(4-Methoxybenzylamino)-5,6,7,8-tetrahydro-benzo[4,5]thieno[2,3-*d*]pyrimidin-2-ylmethoxy]-essigsäure,
[4-(3,4-Dichlorbenzylamino)-5,6,7,8-tetrahydro-benzo[4,5]thieno[2,3-*d*]pyrimidin-2-ylmethoxy]-essigsäure,
[4-(4-Chlorbenzylamino)-5,6,7,8-tetrahydro-benzo[4,5]thieno[2,3-*d*]pyrimidin-2-ylmethoxy]-essigsäure,
[4-(4-Methylbenzylamino)-5,6,7,8-tetrahydro-benzo[4,5]thieno[2,3-*d*]pyrimidin-2-ylmethoxy]-essigsäure,
[4-(4-Fluorbenzylamino)-5,6,7,8-tetrahydro-benzo[4,5]thieno[2,3-*d*]pyrimidin-2-ylmethoxy]-essigsäure,
(4-Benzylamino-5,6,7,8-tetrahydro-benzo[4,5]thieno[2,3-*d*]pyrimidin-2-ylmethoxy)-essigsäure,
[4-(3-Chlor-4-nitrobenzylamino)-5,6,7,8-tetrahydro-benzo[4,5]-thieno[2,3-*d*]pyrimidin-2-ylmethoxy]-essigsäure,
[4-(2,4-Dichlorbenzylamino)-5,6,7,8-tetrahydro-benzo[4,5]thieno[2,3-*d*]pyrimidin-2-ylmethoxy]-essigsäure,
[4-(2-Chlor-4-fluorbenzylamino)-5,6,7,8-tetrahydro-benzo[4,5]-thieno[2,3-*d*]pyrimidin-2-ylmethoxy]-essigsäure,
[4-(3-Fluorbenzylamino)-5,6,7,8-tetrahydro-benzo[4,5]thieno[2,3-*d*]pyrimidin-2-ylmethoxy]-essigsäure,
[4-(2-Methoxybenzylamino)-5,6,7,8-tetrahydro-benzo[4,5]thieno[2,3-*d*]pyrimidin-2-ylmethoxy]-essigsäure,
[4-(2-Chlorbenzylamino)-5,6,7,8-tetrahydro-benzo[4,5]thieno[2,3-*d*]pyrimidin-2-ylmethoxy]-essigsäure,
{4-[3,5-Di-(trifluormethoxy)benzylamino]-5,6,7,8-tetrahydrobenzo[4,5]thieno[2,3-*d*]pyrimidin-2-ylmethoxy}-essigsäure,
[4-(3-Chlor-4-methoxy-benzylamino)-5,6,7,8-tetrahydrobenzo[4,5]thieno[2,3-*d*]pyrimidin-2-ylethoxy]-essigsäure, Ethanolaminsalz, 139-140°;
[4-(3,4-Methylendioxy-benzylamino)-5,6,7,8-tetrahydro-benzo[4,5]-thieno[2,3-*d*]pyrimidin-2-ylethoxy]-essigsäure,
[4-(3,4-Dimethoxy-benzylamino)-5,6,7,8-tetrahydro-benzo[4,5]-thieno[2,3-*d*]pyrimidin-2-ylethoxy]-essigsäure,
[4-(4-Methoxybenzylamino)-5,6,7,8-tetrahydro-benzo[4,5]thieno[2,3-*d*]pyrimidin-2-ylethoxy]-essigsäure,
[4-(3,4-Dichlorbenzylamino)-5,6,7,8-tetrahydro-benzo[4,5]thieno[2,3-*d*]pyrimidin-2-ylethoxy]-essigsäure,
[4-(4-Chlorbenzylamino)-5,6,7,8-tetrahydro-benzo[4,5]thieno[2,3-*d*]pyrimidin-2-ylethoxy]-essigsäure,
[4-(4-Methylbenzylamino)-5,6,7,8-tetrahydro-benzo[4,5]thieno[2,3-*d*]pyrimidin-2-ylethoxy]-essigsäure,
[4-(4-Fluorbenzylamino)-5,6,7,8-tetrahydro-benzo[4,5]thieno[2,3-*d*]pyrimidin-2-ylethoxy]-essigsäure,
(4-Benzylamino-5,6,7,8-tetrahydro-benzo[4,5]thieno[2,3-*d*]pyrimidin-2-ylethoxy)-essigsäure,
[4-(3-Chlor-4-nitrobenzylamino)-5,6,7,8-tetrahydro-benzo[4,5]-thieno[2,3-*d*]pyrimidin-2-ylethoxy]-essigsäure,
[4-(2,4-Dichlorbenzylamino)-5,6,7,8-tetrahydro-benzo[4,5]thieno[2,3-d]pyrimidin-2-ylethoxy]-essigsäure,
[4-(2-Chlor-4-fluorbenzylamino)-5,6,7,8-tetrahydro-benzo[4,5]-thieno[2,3-*d*]pyrimidin-2-ylethoxy]-essigsäure,
[4-(3-Fluorbenzylamino)-5,6,7,8-tetrahydro-benzo[4,5]thieno[2,3-*d*]pyrimidin-2-ylethoxy]-essigsäure,
[4-(2-Methoxybenzylamino)-5,6,7,8-tetrahydro-benzo[4,5]thieno[2,3-d]pyrimidin-2-ylethoxy]-essigsäure,
[4-(2-Chlorbenzylamino)-5,6,7,8-tetrahydro-benzo[4,5]thieno[2,3-*d*]pyrimidin-2-ylethoxy]-essigsäure,
{4-[3,5-Di-(trifluormethoxy)benzylamino]-5,6,7,8-tetrahydrobenzo[4,5]thieno[2,3-*d*]pyrimidin-2-ylethoxy}-essigsäure.

### Beispiel 7

Analog Beispiel 6 erhält man durch Esterspaltung mit NaOH in Methanol aus den in Beispiel 4 und 5 erhaltenen Ethylesterderivaten die nachstehenden Carbonsäurederivate
[4-(3-Chlor-4-methoxy-benzylamino)-5,6,7,8-tetrahydro-benzo[4,5]-thieno[2,3-*d*]pyrimidin-2-ylmethylsulfanyl]-essigsäure, Ethanolaminsatz, F. 161-162°;
[4-(3,4-Methylendioxy-benzylamino)5,6,7,8-tetrahydro-benzo[4,5]-thieno[2,3-*d*]pyrimidin-2-ylmethylsulfanyl]-essigsäure,
[4-(3,4-Dimethoxy-benzylamino)-5,6,7,8-tetrahydro-benzo[4,5]-thieno[2,3-*d*]pyrimidin-2-ylmethylsulfanyl]-essigsäure,
[4-(4-Methoxybenzylamino)-5,6,7,8-tetrahydro-benzo[4,5]thieno[2,3-*d*]pyrimidin-2-ylmethylsulfanyl]-essigsäure,
[4-(3,4-Dichlorbenzylamino)-5,6,7,8-tetrahydro-benzo[4,5]thieno[2,3-*d*]pyrimidin-2-ylmethylsulfanyl]-essigsäure,
[4-(4-Chlorbenzylamino)-5,6,7,8-tetrahydro-benzo[4,5]thieno[2,3-*d*]pyrimidin-2-ylmethylsulfanyl]-essigsäure,
[4-(4-Methylbenzylamino)-5,6,7,8-tetrahydro-benzo[4,5]thieno[2,3-*d*]pyrimidin-2-ylmethylsulfanyl]-essigsäure,
[4-(4-Fluorbenzylamino)-5,6,7,8-tetrahydro-benzo[4,5]thieno[2,3-*d*]pyrimidin-2-ylmethylsulfanyl]-essigsäure,
(4-Benzylamino-5,6,7,8-tetrahydro-benzo[4,5]thieno[2,3-*d*]pyrimidin-2-ylmethylsulfanyl)-essigsäure,
[4-(3-Chlor-4-nitrobenzylamino)-5,6,7,8-tetrahydro-benzo[4,5]-thieno[2,3-*d*]pyrimidin-2-ylmethylsulfanyl]-essigsäureester,
[4-(2,4-Dichlorbenzylamino)-5,6,7,8-tetrahydro-benzo[4,5]thieno[2,3-*d*]pyrimidin-2-ylmethylsulfanyl]-essigsäure,
[4-(2-Chlor-4-fluorbenzylamino)-5,6,7,8-tetrahydro-benzo[4,5]-thieno[2,3-*d*]pyrimidin-2-ylmethylsulfanyl]-essigsäure,
[4-(3-Fluorbenzylamino)-5,6,7,8-tetrahydro-benzo[4,5]thieno[2,3-*d*]pyrimidin-2-ylmethylsulfanyl]-essigsäure,
[4-(2-Methoxybenzylamino)-5,6,7,8-tetrahydro-benzo[4,5]thieno[2,3-*d*]pyrimidin-2-ylmethylsulfanyl]-essigsäure,
[4-(2-Chlorbenzylamino)-5,6,7,8-tetrahydro-benzo[4,5]thieno[2,3-*d*]pyrimidin-2-ylmethylsulfanyl]-essigsäure,
{4-[3,5-Di-(trifluormethoxy)benzylamino]-5,6,7,8-tetrahydrobenzo[4,5]thieno[2,3-*d*]pyrimidin-2-ylmethylsulfanyl}-essigsäure,
[4-(3-Chlor-4-methoxy-benzylamino)-5,6,7,8-tetrahydro-benzo[4,5]-thieno[2,3-*d*]pyrimidin-2-ylmethylsulfinyl]-essigsäure, Ethanolaminsalz, amorph;
[4-(3,4-Methylendioxy-benzylamino)-5,6,7,8-tetrahydro-benzo[4,5]-thieno[2,3-*d*]pyrimidin-2-ylmethylsulfinyl]-essigsäure,
[4-(3,4-Dimethoxy-benzylamino)-5,6,7,8-tetrahydro-benzo[4,5]-thieno[2,3-*d*]pyrimidin-2-ylmethylsulfinyl]-essigsäure,
[4-(4-Methoxybenzylamino)-5,6,7,8-tetrahydro-benzo[4,5]thieno[2,3-*d*]pyrimidin-2-ylmethylsulfinyl]-essigsäure,
[4-(3,4-Dichlorbenzytamino)-5,6,7,8-tetrahydro-benzo[4,5]thieno[2,3-*d*]pyrimidin-2-ylmethylsulfinyl]-essigsäure,
[4-(4-Chlorbenzylamino)-5,6,7,8-tetrahydro-benzo[4,5]thieno[2,3-*d*]pyrimidin-2-ylmethylsulfinyl]-essigsäure,
[4-(4-Methylbenzylamino)-5,6,7,8-tetrahydro-benzo[4,5]thieno[2,3-*d*]pyrimidin-2-ylmethylsulfinyl]-essigsäure,
[4-(4-Fluorbenzylamino)-5,6,7,8-tetrahydro-benzo[4,5]thieno[2,3-*d*]pyrimidin-2-ylmethylsulfinyl]-essigsäure,
(4-Benzylamino-5,6,7,8-tetrahydro-benzo[4,5]thieno[2,3-*d*]pyrimidin-2-ylmethylsulfinyl)-essigsäure,
[4-(3-Chlor-4-nitrobenzylamino)-5,6,7,8-tetrahydro-benzo[4,5]-thieno[2,3-*d*]pyrimidin-2-ylmethylsulfinyl]-essigsäure,
[4-(2,4-Dichlorbenzylamino)-5,6,7,8-tetrahydro-benzo[4,5]thieno[2,3-*d*]pyrimidin-2-ylmethylsulfinyl)-essigsäure,
[4-(2-Chlor-4-fluorbenzylamino)-5,6,7,8-tetrahydro-benzo[4,5]-thieno[2,3-*d*]pyrimidin-2-ylmethylsulfinyl]-essigsäure,
[4-(3-Fluorbenzylamino)-5,6,7,8-tetrahydro-benzo[4,5]thieno[2,3-*d*]pyrimidin-2-ylmethylsulfinyl]-essigsäure,
[4-(2-Methoxybenzylamino)-5,6,7,8-tetrahydro-benzo[4,5]thieno[2,3-*d*]pyrimidin-2-ylmethylsulfinyl]-essigsäure,
[4-(2-Chlorbenzylanlino)-5,6,7,8-tetrahydro-benzo[4,5]thieno[2,3-*d*]pyrimidin-2-ylmethylsulfinyl]-essigsäure,
{4-[3,5-Di-(trifluormethoxy)benzylamino]-5,6,7,8-tetrahydrobenzo[4,5]thieno[2,3-*d*]pyrimidin-2-ylmethylsulfinyl}-essigsäure.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ - 12 H₂O und 0,1 g Benzalkoniumchlorid in 940 ml zweifach destilliertem Wasser. Man stellt auf pH 6,8 ein, füllt auf 1 auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g Wirkstoff der Formel I in 10 l isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Verbindungen der Formel I 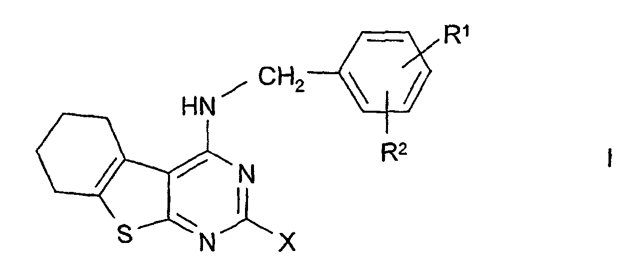 worin
R¹, R² jeweils unabhängig voneinander H, A, OH, OA, NO₂ o- der Hal,
R¹ und R² zusammen auch Alkylen mit 3-5 C-Atomen, -O-CH₂-CH₂-, -CH₂-O-CH₂-, -O-CH₂-O- oder -O-CH₂-CH₂-O-,
X einfach durch R⁵ substituiertes R³ oder R⁴,
R³ lineares oder verzweigtes Alkylen mit 1-10 C-Atomen, worin eine oder zwei CH₂-Gruppen durch -CH=CH-Gruppen, O, NH oder NA ersetzt sein können,
R⁴ Cycloalkyl oder Cycloalkylalkylen mit 5-12 C-Atomen,
R⁵ O(CH₂)ₙCOOH, O(CH₂)ₙCOOA, O(CH₂)ₙCONH₂, O(CH₂)ₙCONHA, O(CH₂)ₙCONA₂ oder O(CH₂)ₙCN, S(O)ₘ(CH₂)ₙCOOH, S(O)ₘ(CH₂)ₙCOOA, S(O)ₘ(CH₂)ₙCONH₂, S(O)ₘ(CH₂)ₙCONHA, S(O)ₘ(CH₂)ₙCONA₂ oder S(O)ₘ(CH₂)ₙCN,
m 0, 1 oder 2,
n 1 oder 2,
A Alkyl mit 1 bis 6 C-Atomen und
Hal F, Cl, Br oder I
bedeuten,
sowie deren physiologisch unbedenklichen Salze und/oder Solvate.

2. Verbindungen nach Anspruch 1
worin
X durch O(CH₂)ₙCOOH, O(CH₂)ₙCOOA, O(CH₂)ₙCONH₂, O(CH₂)ₙCONHA, O(CH₂)ₙCONA₂ oder O(CH₂)ₙCN, S(O)ₘ(CH₂)ₙCOOH, S(O)ₘ(CH₂)ₙCOOA, S(O)ₘ(CH₂)ₙCONH₂, S(O)ₘ(CH₂)ₙCONHA, S(O)ₘ(CH₂)ₙCONA₂ oder S(O)ₘ(CH₂)ₙCN substituiertes R³
bedeutet,
sowie deren physiologisch unbedenklichen Salze und/oder Solvate.

3. Verbindungen nach Anspruch 1
worin
R¹, R² jeweils unabhängig voneinander Hal, OH oder OA,
X durch O(CH₂)ₙCOOH, O(CH₂)ₙCOOA, S(O)ₘ(CH₂)ₙCOOH, S(O)ₘ(CH₂)ₙCOOA, substituiertes R³
bedeuten,
sowie deren physiologisch unbedenklichen Salze und/oder Solvate.

4. Verbindungen nach Anspruch 1
worin
R¹, R² jeweils unabhängig voneinander Hal, OH oder OA,
X durch O(CH₂)ₙCOOH, O(CH₂)ₙCOOA, S(O)ₘ(CH₂)ₙCOOH, S(O)ₘ(CH₂)ₙCOOA, substituiertes R³,
R³ Methylen, Ethylen oder Propylen
bedeuten,
sowie deren physiologisch unbedenklichen Salze und/oder Solvate.

5. Verbindungen nach Anspruch 1
worin
R¹, R² jeweils unabhängig voneinander Hal, OH oder OA,
R¹ und R² zusammen Alkylen mit 3-5 C-Atomen, -O-CH₂-CH₂-, -O-CH₂O- oder -O-CH₂-CH₂-O,
X durch O(CH₂)ₙCOOH, O(CH₂)ₙCOOA, S(O)ₘ(CH₂)ₙCOOH, S(O)ₘ(CH₂)ₙCOOA, substituiertes R³,
R³ Methylen, Ethylen oder Propylen
bedeuten,
sowie deren physiologisch unbedenklichen Salze und/oder Solvate.

6. Verbindungen nach Anspruch 1
worin
R¹, R² jeweils unabhängig voneinander H, Hal, A, NO₂, OH o- der OA,
R¹ und R² zusammen Alkylen mit 3-5 C-Atomen, -O-CH₂-CH₂-, -O-CH₂-O- oder -O-CH₂-CH₂-O-,
X durch O(CH₂)ₙCOOH, O(CH₂)ₙCOOA, S(O)ₘ(CH₂)ₙCOOH, S(O)ₘ(CH₂)ₙCOOA, substituiertes R³,
R³ Methylen, Ethylen oder Propylen,
A Alkyl mit 1-6 C-Atomen oder CF₃
bedeuten,
sowie deren physiologisch unbedenklichen Salze und/oder Solvate.

7. Verbindungen nach Anspruch 1
worin
R¹, R² jeweils unabhängig voneinander H, Hal, A, NO₂, OH o- der OA,
R¹ und R² zusammen -O-CH₂-O- oder -O-CH₂-CH₂-O-,
X durch O(CH₂)ₙCOOH, O(CH₂)ₙCOOA, S(O)ₘ(CH₂)ₙCOOH, S(O)ₘ(CH₂)ₙCOOA, substituiertes R³,
R³ Methylen, Ethylen oder Propylen,
A Alkyl mit 1-6 C-Atomen oder CF₃
bedeuten,
sowie deren physiologisch unbedenklichen Salze und/oder Solvate.

8. Verbindungen gemäß Anspruch 1
(a) 2-[4-(3-Chlor-4-methoxy-benzylamino)-5,6,7,8-tetrahydro-[4,5]-benzothieno-[2,3-*d*]-pyrimidin-2-ylmethoxy]-essigsäure;
(b) 2-[4-(3-Chlor-4-methoxy-benzylamino)-5,6,7,8-tetrahydro-[4,5]-benzothieno-[2,3-*d*]-pyrimidin-2-ylmethylsulfanyl]-essigsäure;
(c) 2-[4-(3-Chlor-4-methoxy-benzylamino)-5,6,7,8-tetrahydro-[4,5]-benzothieno-[2,3-*d*]-pyrimidin-2-ylmethylsulfinyl]-essigsäure;
(d) 2-[4-(3-Chlor-4-methoxy-benzylamino)-5,6,7,8-tetrahydro-[4,5]-benzothieno-[2,3-*d*]-pyrimidin-2-ylethoxy]-essigsäure;
sowie deren physiologisch unbedenklichen Salze und/oder Solvate.

9. Verfahren zur Herstellung
von Verbindungen der Formel I nach Anspruch 1 sowie deren Salzen,
**dadurch gekennzeichnet, daß** man
a) eine Verbindung der Formel II 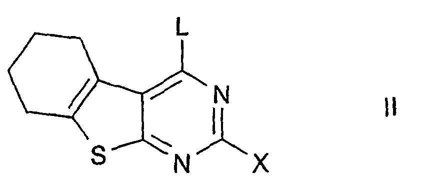 worin
X die angegebene Bedeutung hat,
und L Cl, Br, OH, SCH₃ oder eine reaktionsfähige veresterte OH-Gruppe bedeutet,
mit einer Verbindung der Formel III 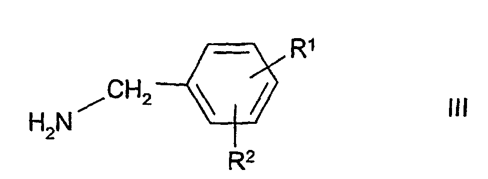 worin
R¹ und R² die angegebenen Bedeutungen haben,
umsetzt,
oder
b) in einer Verbindung der Formel I einen Rest X in einen anderen Rest X umwandelt, indem man z.B. eine Estergruppe zu einer COOH-Gruppe hydrolysiert oder eine COOH-Gruppe in ein Amid oder in eine Cyangruppe umwandelt
und/oder daß man eine Verbindung der Formel I in eines ihrer Salze überführt.

10. Verbindungen der Formel I gemäß der Ansprüche 1 bis 8 sowie deren physiologisch unbedenklichen Salze und Solvate als Arzneimittel.

11. Arzneimittel nach Anspruch 10 zur Inhibierung der Phosphodiesterase V.

12. Arzneimittel nach Anspruch 10 oder 11 zur Bekämpfung von Krankheiten des Herz-Kreislaufsystems und zur Behandlung und/oder Therapie von Potenzstörungen.

13. Arzneimittel nach Anspruch 10, 11 oder 12 zur Behandlung von Angina, Bluthochdruck, pulmonarem Hochdruck, congestivem Herzversagen, Atherosklerose, Bedingungen verminderter Durchgängigkeit der Herzgefäße, peripheren vaskulären Krankheiten, Schlaganfall, Bronchitis, allergischem Asthma, chronischem Asthma, allergischer Rhinitis, Glaucom, Irritable Bowel Syndrome, Tumoren, Niereninsuffizienz, Leberzirrhose und zur Behandlung weiblicher Sexualstörungen.

14. Pharmazeutische Zubereitung, enthaltend mindestens ein Arzneimittel gemäß einem der Ansprüche 10 bis 13 sowie gegebenenfalls Träger- und/oder Hilfsstoffe und gegebenenfalls andere Wirkstoffe.

15. Verwendung von Verbindungen gemäß der Ansprüche 1 bis 8 und/oder ihrer physiologisch unbedenklichen Salze oder Solvate zur Herstellung eines Arzneimittels zur Bekämpfung von Krankheiten des Herz-Kreislaufsystems und zur Behandlung und/oder Therapie von Potenzstörungen.

16. Verwendung von Verbindungen gemäß der Ansprüche 1 bis 8 und/oder ihrer physiologisch unbedenklichen Salze oder Solvate zur Herstellung eines Arzneimittels zur Behandlung von Angina, Bluthochdruck, pulmonarem Hochdruck, congestivem Herzversagen, Atherosklerose, Bedingungen verminderter Durchgängigkeit der Herzgefäße, peripheren vaskulären Krankheiten, Schlaganfall, Bronchitis, allergischem Asthma, chronischem Asthma, allergischer Rhinitis, Glaucom, Irritable Bowel Syndrome, Tumoren, Niereninsuffizienz, Leberzirrhose und zur Behandlung weiblicher Sexualstörungen.

## Claims

1. Compounds of the formula I 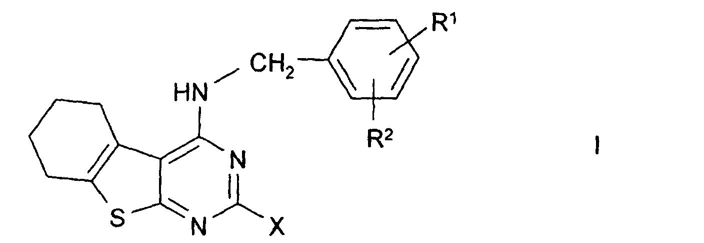 in which
R¹, R² each, independently of one another, denote H, A, OH, OA, NO₂ or Hal,
R¹ and R² together also denote alkylene having 3-5 C atoms, -O-CH₂-CH₂-, -CH₂-O-CH₂-, -O-CH₂-O- or -O-CH₂-CH₂-O-,
X denotes mono-R⁵-substituted R³ or R⁴,
R³ denotes linear or branched alkylene having 1-10 C atoms, in which one or two CH₂ groups may be replaced by -CH=CH- groups, O, NH or NA,
R⁴ denotes cycloalkyl or cycloalkylalkylene having 5-12 C atoms,
R⁵ denotes O(CH₂)ₙCOOH, O(CH₂)ₙCOOA, O(CH₂)ₙCONH₂, O(CH₂)ₙCONHA, O(CH₂)ₙCONA₂ or O(CH₂)ₙCN, S(O)ₘ(CH₂)ₙCOOH, S(O)ₘ(CH₂)ₙCOOA, S(O)ₘ(CH₂)ₙCONH₂, S(O)ₘ(CH₂)ₙCONHA, S(O)ₘ(CH₂)ₙCONA₂ or S(O)ₘ(CH₂)ₙCN,
m denotes 0, 1 or 2,
n denotes 1 or 2,
A denotes alkyl having 1 to 6 C atoms and
Hal denotes F, Cl, Br or I,
and physiologically acceptable salts and/or solvates thereof.

2. Compounds according to Claim 1
in which
X denotes R³ which is substituted by O(CH₂)ₙCOOH, O(CH₂)ₙCOOA, O(CH₂)ₙCONH₂, O(CH₂)ₙCONHA, O(CH₂)ₙCONA₂ or O(CH₂)ₙCN, S(O)ₘ(CH₂)ₙCOOH, S(O)ₘ(CH₂)ₙCOOA, S(O)ₘ(CH₂)ₙCONH₂, S(O)ₘ(CH₂)ₙCONHA, S(O)ₘ(CH₂)ₙCONA₂ or S(O)ₘ(CH₂)ₙCN,
and physiologically acceptable salts and/or solvates thereof.

3. Compounds according to Claim 1
in which
R¹, R² each, independently of one another, denote Hal, OH or OA,
X denotes R³ which is substituted by O(CH₂)ₙCOOH, O(CH₂)ₙCOOA, S(O)ₘ(CH₂)ₙCOOH, S(O)ₘ(CH₂)ₙCOOA,
and physiologically acceptable salts and/or solvates thereof.

4. Compounds according to Claim 1
in which
R¹, R² each, independently of one another, denote Hal, OH or OA,
X denotes R³ which is substituted by O(CH₂)ₙCOOH, O(CH₂)ₙCOOA, S(O)ₘ(CH₂)ₙCOOH, S(O)ₘ(CH₂)ₙCOOA,
R³ denotes methylene, ethylene or propylene,
and physiologically acceptable salts and/or solvates thereof.

5. Compounds according to Claim 1
in which
R¹, R² each, independently of one another, denote Hal, OH or OA,
R¹ and R² together denote alkylene having 3-5 C atoms, -O-CH₂-CH₂-, -O-CH₂-O- or -O-CH₂-CH₂-O,
X denotes R³ which is substituted by O(CH₂)ₙCOOH, O(CH₂)ₙCOOA, S(O)ₘ(CH₂)ₙCOOH, S(O)ₘ(CH₂)ₙCOOA,
R³ denotes methylene, ethylene or propylene,
and physiologically acceptable salts and/or solvates thereof.

6. Compounds according to Claim 1
in which
R¹, R² each, independently of one another, denote H, Hal, A, NO₂, OH or OA,
R¹ and R² together denote alkylene having 3-5 C atoms, -O-CH₂-CH₂-, -O-CH₂-O- or -O-CH₂-CH₂-O,
X denotes R³ which is substituted by O(CH₂)ₙCOOH, O(CH₂)ₙCOOA, S(O)ₘ(CH₂)ₙCOOH, S(O)ₘ(CH₂)ₙCOOA,
R³ denotes methylene, ethylene or propylene,
A denotes alkyl having 1-6 C atoms or CF₃,
and physiologically acceptable salts and/or solvates thereof.

7. Compounds according to Claim 1
in which
R¹, R² each, independently of one another, denote H, Hal, A, NO₂, OH or OA,
R¹ and R² together denote -O-CH₂-O- or -O-CH₂-CH₂-O-,
X denotes R³ which is substituted by O(CH₂)ₙCOOH, O(CH₂)ₙCOOA, S(O)ₘ(CH₂)ₙCOOH, S(O)ₘ(CH₂)ₙCOOA,
R³ denotes methylene, ethylene or propylene,
A denotes alkyl having 1-6 C atoms or CF₃,
and physiologically acceptable salts and/or solvates thereof.

8. Compounds according to Claim 1
(a) 2-[4-(3-chloro-4-methoxybenzylamino)-5,6,7,8-tetrahydro[4,5]-benzothieno[2,3-*d*]pyrimidin-2-ylmethoxy]acetic acid;
(b) 2-[4-(3-chloro-4-methoxybenzylamino)-5,6,7,8-tetrahydro[4,5]-benzothieno[2,3-*d*]pyrimidin-2-ylmethylsulfanyl]acetic acid;
(c) 2-[4-(3-chloro-4-methoxybenzylamino)-5,6,7,8-tetrahydro[4,5]-benzothieno[2,3-*d*]pyrimidin-2-ylmethylsulfinyl]acetic acid;
(d) 2-[4-(3-chloro-4-methoxybenzylamino)-5,6,7,8-tetrahydro[4,5]-benzothierro[2,3-*d*]pyrimidin-2-ylethoxy]acetic acid;
and physiologically acceptable salts and/or solvates thereof.

9. Process for the preparation
of compounds of the formula I according to Claim 1 and salts thereof,
**characterised in that**
a) a compound of the formula II 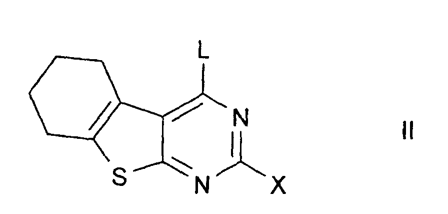 in which
X has the meaning indicated,
and L denotes Cl, Br, OH, SCH₃ or a reactive esterified OH group,
is reacted with a compound of the formula III 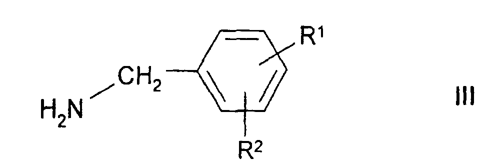 in which
R¹ and R² have the meanings indicated,
or
b) a radical X in a compound of the formula I is converted into another radical X by, for example, hydrolysing an ester group to a COOH group or converting a COOH group into an amide or into a cyano group,
and/or **in that** a compound of the formula I is converted into one of its salts.

10. Compounds of the formula I according to Claims 1 to 8 and physiologically acceptable salts and solvates thereof as medicaments.

11. Medicaments according to Claim 10 for the inhibition of phosphodiesterase V.

12. Medicaments according to Claim 10 or 11 for combating diseases of the cardiovascular system and for the treatment and/or therapy of potency disorders.

13. Medicaments according to Claim 10, 11 or 12 for the treatment of angina, high blood pressure, high pulmonary pressure, congestive heart failure, atherosclerosis, conditions of reduced passage through the heart vessels, peripheral vascular diseases, strokes, bronchitis, allergic asthma, chronic asthma, allergic rhinitis, glaucoma, irritable bowel syndrome, tumours, renal insufficiency, liver cirrhosis and for the treatment of female sexual disorders.

14. Pharmaceutical composition comprising at least one medicament according to one of Claims 10 to 13 and optionally excipients and/or adjuvants and optionally other active ingredients.

15. Use of compounds according to Claims 1 to 8 and/or physiologically acceptable salts or solvates thereof for the preparation of a medicament for combating diseases of the cardiovascular system and for the treatment and/or therapy of potency disorders.

16. Use of compounds according to Claims 1 to 8 and/or physiologically acceptable salts or solvates thereof for the preparation of a medicament for the treatment of angina, high blood pressure, high pulmonary pressure, congestive heart failure, atherosclerosis, conditions of reduced passage through the heart vessels, peripheral vascular diseases, strokes, bronchitis, allergic asthma, chronic asthma, allergic rhinitis, glaucoma, irritable bowel syndrome, tumours, renal insufficiency, liver cirrhosis and for the treatment of female sexual disorders.

## Revendications

1. Composés de la formule I 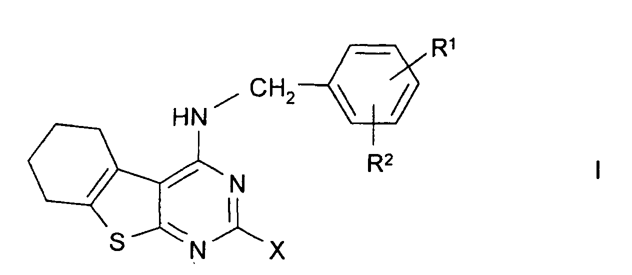 dans laquelle
R¹, R² chacun indépendamment l'un de l'autre, représentent H, A, OH, OA, NO₂ ou Hal,
R¹ et R² ensemble représentent également alkylène comportant de 3 à 5 atomes de C, -O-CH₂-CH₂-, -CH₂-O-CH₂-, -O-CH₂-O- ou -O-CH₂-CH₂-O-,
X représente R³ ou R⁴ mono-R⁵-substitué,
R³ représente alkylène linéaire ou ramifié comportant de 1 à 10 atomes de C, où un ou deux groupes CH₂ peuvent être remplacés par des groupes -CH=CH-, O, NH ou NA,
R⁴ représente cycloalkyle ou cycloalkylalkylène comportant de 5 à 12 atomes de C,
R⁵ représente O(CH₂)ₙCOOH, O(CH₂)ₙCOOA, O(CH₂)ₙCONH₂, O(CH₂)ₙCONHA, O(CH₂)ₙCONA₂ ou O(CH₂)ₙCN, S(O)ₘ(CH₂)ₙCOOH, S(O)ₘ(CH₂)ₙCOOA, S(O)ₘ(CH₂)ₙCONH₂, S(O)ₘ(CH₂)ₙCONHA, S(O)ₘ(CH₂)ₙCONA₂ ou S(O)ₘ(CH₂)ₙCN,
m représente 0, 1 ou 2,
n représente 1 ou 2,
A représente alkyle comportant de 1 à 6 atomes de C et
Hal représente F, Cl, Br ou I,
et leurs sels et/ou solvates physiologiquement acceptables.

2. Composés selon la revendication 1
dans lesquels
X représente R³ qui est substitué par O(CH₂)ₙCOOH, O(CH₂)ₙCOOA, O(CH₂)ₙCONH₂, O(CH₂)ₙCONHA, O(CH₂)ₙCONA₂ ou O(CH₂)ₙCN, S(O)ₘ(CH₂)ₙCOOH, S(O)ₘ(CH₂)ₙCOOA, S(O)ₘ(CH₂)ₙCONH₂, S(O)ₘ(CH₂)ₙCONHA, S(O)ₘ(CH₂)ₙCONA₂ ou S(O)ₘ(CH₂)ₙCN,
et leurs sels et/ou solvates physiologiquement acceptables.

3. Composés selon la revendication 1
dans lesquels
R¹, R² chacun indépendamment l'un de l'autre, représentent Hal, OH ou OA,
X représente R³ qui est substitué par O(CH₂)ₙCOOH, O(CH₂)ₙCOOA, S(O)ₘ(CH₂)ₙCOOH, S(O)ₘ(CH₂)ₙCOOA,
et leurs sels et/ou solvates physiologiquement acceptables.

4. Composés selon la revendication 1
dans lesquels
R¹, R² chacun indépendamment l'un de l'autre, représentent Hal, OH ou OA,
X représente R³ qui est substitué par O(CH₂)ₙCOOH,
O(CH₂)ₙCOOA, S(O)ₘ(CH₂)ₙCOOH, S(O)ₘ(CH₂)ₙCOOA,
R³ représente méthylène, éthylène ou propylène,
et leurs sels et/ou solvates physiologiquement acceptables.

5. Composés selon la revendication 1
dans lesquels
R¹, R² chacun indépendamment l'un de l'autre, représentent Hal, OH ou OA,
R¹ et R² ensemble représentent alkylène comportant de 3 à 5 atomes de C, -O-CH₂-CH₂-, -O-CH₂-O- ou -O-CH₂-CH₂-O,
X représente R³ qui est substitué par O(CH₂)ₙCOOH,
O(CH₂)ₙCOOA, S(O)ₘ(CH₂)ₙCOOH, S(O)ₘ(CH₂)ₙCOOA,
R³ représente méthylène, éthylène ou propylène,
et leurs sels et/ou solvates physiologiquement acceptables.

6. Composés selon la revendication 1
dans lesquels
R¹, R² chacun indépendamment l'un de l'autre, représentent H, Hal, A, NO₂, OH ou OA,
R¹ et R² ensemble représentent alkylène comportant de 3 à 5 atomes de C, -O-CH₂-CH₂-, -O-CH₂-O- ou -O-CH₂-CH₂-O,
X représente R³ qui est substitué par O(CH₂)ₙCOOH,
O(CH₂)ₙCOOA, S(O)ₘ(CH₂)ₙCOOH, S(O)ₘ(CH₂)ₙCOOA,
R³ représente méthylène, éthylène ou propylène,
A représente alkyle comportant de 1 à 6 atomes de C ou CF₃,
et leurs sels et/ou solvates physiologiquement acceptables.

7. Composés selon la revendication 1
dans lesquels
R¹, R² chacun indépendamment l'un de l'autre, représentent H, Hal, A, NO₂, OH ou OA,
R¹ et R² ensemble représentent -O-CH₂-O- ou -O-CH₂-CH₂-O-,
X représente R³ qui est substitué par O(CH₂)ₙCOOH, O(CH₂)ₙCOOA, S(O)ₘ(CH₂)ₙCOOH, S(O)ₘ(CH₂)ₙCOOA,
R³ représente méthylène, éthylène ou propylène,
A représente alkyle comportant de 1 à 6 atomes de C ou CF₃,
et leurs sels et/ou solvates physiologiquement acceptables.

8. Composés selon la revendication 1
(a) acide 2-[4-(3-chloro-4-méthoxybenzylamino)-5,6,7,8-tétrahydro-[4,5]benzothiéno[2,3-*d*]pyrimidine-2-ylméthoxy]acétique;
(b) acide 2-[4-(3-chloro-4-méthoxybenzylamino)-5,6,7,8-tétrahydro-[4,5]benzothiéno[2,3-*d*]pyrimidine-2-ylméthylsulfanyl]acétique;
(c) acide 2-[4-(3-chloro-4-méthoxybenzylamino)-5,6,7,8-tétrahydro-[4,5]benzothiéno[2,3-*d*]pyrimidine-2-ylméthylsulfinyl]acétique;
(d) acide 2-[4-(3-chloro-4-méthoxybenzylamino)-5,6,7,8-tétrahydro-[4,5]benzothiéno[2,3-*d*]pyrimidine-2-yléthoxy]acétique;
et leurs sels et/ou solvates physiologiquement acceptables.

9. Procédé pour la préparation
de composés de la formule I selon la revendication 1 et de leurs sels,
**caractérisé en ce que**
a) un composé de la formule II 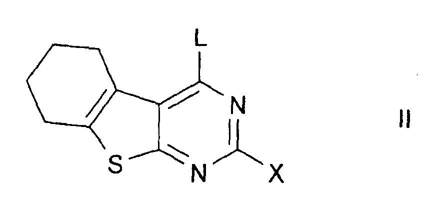 dans laquelle
X présente la signification indiquée,
et L représente CI, Br, OH, SCH₃ ou un groupe OH estérifié réactif,
est amené à réagir avec un composé de la formule III 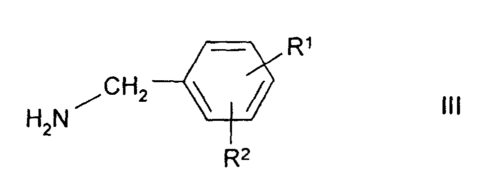 dans laquelle
R¹ et R² présentent les significations indiquées,
ou
b) un radical X dans un composé de la formule I est converti selon un autre radical X par, par exemple, hydrolyse d'un groupe ester selon un groupe COOH ou par conversion d'un groupe COOH selon un amide ou selon un groupe cyano,
et/ou **en ce qu'**un composé de la formule I est converti selon l'un de ses sels.

10. Composés de la formule 1 selon les revendications 1 à 8 et leurs sels et/ou solvates physiologiquement acceptables en tant que médicaments.

11. Médicaments selon la revendication 10 pour l'inhibition de la phosphodiestérase V.

12. Médicaments selon la revendication 10 ou 11 pour combattre des maladies du système cardiovasculaire et pour le traitement et/ou la thérapie de troubles de la puissance sexuelle.

13. Médicaments selon la revendication 10, 11 ou 12 pour le traitement de l'angine, de la pression sanguine élevée, de la pression pulmonaire élevée, de la défaillance du coeur par congestion, de l'athérosclérose, des conditions de passage réduit au travers des vaisseaux du coeur, des maladies vasculaires périphériques, des attaques, des bronchites, de l'asthme allergique, de l'asthme chronique, de la rhinite allergique, des glaucomes, du syndrome du côlon irritable, des tumeurs, de l'insuffisance rénale, de la cirrhose du foie et pour le traitement des troubles sexuels de la femme.

14. Composition pharmaceutique comprenant au moins un médicament selon l'une des revendications 10 à 13 et en option, des excipients et/ou adjuvants et en option, d'autres ingrédients actifs.

15. Utilisation de composés selon les revendications 1 à 8 et/ou de leurs sels et/ou solvates physiologiquement acceptables pour la préparation d'un médicament pour combattre les maladies du système cardiovasculaire et pour le traitement et/ou la thérapie de troubles de la puissance sexuelle.

16. Utilisation de composés selon les revendications 1 à 8 et/ou de leurs sels et/ou solvates physiologiquement acceptables pour la préparation d'un médicament pour le traitement de l'angine, de la pression sanguine élevée, de la pression pulmonaire élevée, de la défaillance du coeur par congestion, de l'athérosclérose, des conditions de passage réduit au travers des vaisseaux du coeur, des maladies vasculaires périphériques, des attaques, de la bronchite, de l'asthme allergique, de l'asthme chronique, de la rhinite allergique, des glaucomes, du syndrome du côlon irritable, des tumeurs, de l'insuffisance rénale, de la cirrhose du foie et pour le traitement des troubles sexuels de la femme.
